# EUROPEAN PATENT SPECIFICATION

(11) **EP 1 119 770 B1**
(45) Date of publication and mention of the grant of the patent: **02.01.2003**
(21) Application number: 99947725.0
(22) Date of filing: 01.10.1999
(51) Int. Cl.: G01N 33/543, G01N 33/545, G01N 33/569, G01N 33/94

(54) **PROCESS AND APPARATUS FOR THE IN VITRO DETECTION OF MULTIPLE ANALYTES**
VERFAHREN UND VORRICHTUNG ZUM IN-VITRO NACHWEIS VON MEHREREN ANALYTEN
PROCEDE ET APPAREIL DESTINES A LA DETECTION IN VITRO DE SUBSTANCES MULTIPLES

(30) Priority: 02.10.1998 GB 9821548
(43) Date of publication of application: 01.08.2001
(73) Proprietor: ABP Diagnostics Ltd., Stone, Staffordshire ST15 8XU (GB)
(72) Inventor: PATEL, Chandravadan, Laguna Niguel, CA 92677 (US)
(74) Representative: Beresford, Keith Denis Lewis
(86) International application number: GB9903261
(87) International publication number: WO00020862

(56) References cited:
- EP-A- 0 319 294
- EP-A- 0 402 023
- EP-A- 0 537 827
- WO-A-85/05451
- WO-A-93/06486
- WO-A-94/14069
- WO-A-96/12965
- WO-A-97/32213
- WO-A-98/13519
- US-A- 5 096 809

## Description

### FIELD OF THE INVENTION

The present invention provides a process and apparatus for the rapid *in vitro* detection of antigens, antibodies, or both or for the detection of small molecules e.g. hormones, pharmaceuticals or drugs of abuse. The test method may be applied to samples derived from birds or mammals, including human beings.

### BACKGROUND TO THE INVENTION

US-A-4407943 is concerned with the detection of an antigen or antibody using immunochemical reactions, and explains that samples of a bodily fluid can be tested for the presence of a particular antigen or antibody, from which a diagnostic inference may be made. The immunochemically reactive antigen or antibody can be coupled to an insoluble carrier material. For this purpose the interior and external surfaces of a micro-porous membrane are coated with a water-insoluble protein such as zein, collagen, fibrinogen, keratin etc. which renders the membrane capable of immobilising a wide variety of biologically active proteins. The coated membrane retains its properties of permitting liquid flow through the pores. Antigen can be immobilised on the membrane, after which there is also immobilised on the membrane a second protein which is immunochemically neutral in the proposed test (e.g. fetal calf serum or bovine gamma globulin) and which substantially neutralises non-specific binding when the membrane is exposed to a mixture of proteinaceous test materials. In the so-called EL-1 method antibody against an antigen to be detected is immobilised on the membrane which is then exposed to a fluid to be tested for the presence of an antigen. The immobilised antibody then harvests the antigen from the solution by binding it into place. The membrane is then separated from the solution, washed free of contaminants and exposed to a solution of an enzyme-tagged antibody which is a covalent conjugate of an antibody and an enzyme to give the structure

C-Ab-Ag-Ab-Enz

where C symbolises the carrier, Ab symbolises antibody, Ag symbolises antigen and Enz symbolises a conjugated enzyme. The alternative EL-2 assay gives the structure:

C-Ag-Ab-AntiAb-Enz

in which AntiAb-Enz symbolises the conjugate of an antibody with an enzyme. The entire immunochemical reaction occurs on the carrier, and components which become bound to the carrier can be separated from unbound components which are simple to remove.

The apparatus for carrying out the above test comprises a tray and a holder for receiving cups each of which has its lower end in the form of a membrane to which an antibody or antigen is attached and which is heat sealed to the lower rim of the cup.
In use the sample is poured into the cup and flows through the membrane into a tray, and the membrane can be washed and treated with reagents as needed to detect the presence or absence of a particular material in a sample. The pore size of the membrane is selected depending on the requirements of the specific assay including the flow characteristics of the sample to be tested and the amount of fluid to be used in the test Application of fluid dropwise to the upper surface of the membrane causes flow of fluid through the membrane to be governed only by the hydrostatic pressure exerted by the drop as it rests on the top of the membrane. If the drop is small enough so that its size approaches the retention volume of the membrane, the fluid will tend to flow into the pores of the membrane and be retained there by capillary attraction until displaced by additional fluid. Reaction time is then controlled by the residence time of the sample within the membrane pores. The test can produce a visible colour change.

In the above patent, a single antibody or antigen covers the entire surface of the membrane, and there is no disclosure of a membrane material that combines the properties that on the one hand quantities of antibody, antigen or hapten can be deposited from an aqueous solution in selected small areas or spots where they become bound to the membrane to provide localised readable areas without spreading uncontrollably across the membrane and on the other hand that samples and treatment reagents can be made to flow naturally through the membrane. There is a conflict between a desire for large pore size to assist fluid flow through the membrane at an acceptable speed and a desire for small pore size to enable good entrapment to be achieved. Practical embodiments of the above test do not rely on simple gravity flow across the membrane but use a vacuum e.g. from a water pump to give rise to the required liquid flow. No provision can be made for carrying out analysis of multi-analytes.

US-A-4855240 discloses a test device and sandwich assay without separate incubation and wash steps using a nitrocellulose test sheet part of which (and in particular an analyte test area) is visible through a test view window. First and second strips of absorbent material (e.g. glass fibre) provide flow paths from respective sample and developer loading ports to one side of the test sheet, the sample loading port being closer to the test view window than the developer loading point. A piece of blotting paper or other material of high liquid absorption capacity is positioned in contact with the test sheet to receive and store test material. The sample contacts the material on the test sheet before the developer contacts either the binder or the sample. Liquid flow is across the test sheet from one side to the other. However, sensitivity is limited by the limited volume of reagents that can be used in the disclosed flow configuration along the surface of the test sheet. No use of a wash solution is disclosed, probably because of the limited liquid absorption capacity of the blotting paper, and detection of multi-analytes is also not disclosed.

EP-A-0 402 023 discloses an assay device and method for detecting an analyte in a liquid sample derived from biological specimen. The assay device includes (a) a porous reaction membrane with a covalently immobilized receptor, (b) an absorbent material in contact with the reaction membrane, (c) a plurality of reaction sites by immobilizing different antibodies specific for predetermined different antigens on distinct spots on the membrane and (d) means for supply of sample and reagents. There is no disclosure of the presence of a plasma separator which has the advantage that the pretreatment of blood samples can be omitted.

It is an object of the present invention to provide a convenient and versatile process and apparatus for carrying out immunological tests on multi-analytes (including the case where there is a test analyte and a control analyte as well as cases where there is a plurality of test analytes and a control analyte).

It is also an object of the invention to provide a process and apparatus for carrying out immunological tests in which larger volumes of reagents can be used to improve sensitivity.

### SUMMARY OF THE INVENTION

The present invention provides apparatus for performing a multi-analyte immunological test comprising:
(a) a membrane having first and second faces between which aqueous liquid can flow and having a microporous structure permitting aqueous liquid to flow gradually from one face to the other;
(b) a plurality of readable areas spaced apart from one another across the membrane and each having a respective antigen, antibody or hapten immobilised thereon;
(c) a cover for a first face of the membrane and formed with means defining a well for supply of aqueous liquid to the membrane, the readable areas being visible through the well and being arranged for simultaneous contact with aqueous liquid supplied into the well; and
(d) absorbent material in contact with the second face of the membrane for reception of aqueous liquid which has flowed across the membrane, there being tight contact between the first face of the membrane and the well, and between the second face of the membrane and the absorbent material.

The invention also provides a test kit comprising a cassette containing the apparatus as set out above within a housing e.g. of plastics material, and dispensing containers for wash solution and for a solution for developing or visualising the result of the test.

The invention therefore contemplates a method and a system for detecting in a sample (serum, plasma, whole blood, saliva or urine) of human or animal origin the presence or absence of a single or multiple analyte. The detection is simple and rapid. The detection system comprises a flow-through plastic device containing an absorbent pad with an affinity membrane. The device has specific areas of the membrane in which antigenic materials are immobilised, the membrane being of a hydrophobic material so that droplets of aqueous liquid containing the analyte remain localised and resist diffusing away from the place where they have become deposited. Each device has a control substance in one of the areas to act as a quality control material to check that the device is functioning correctly. In the case of antibody detection, the control material used is normally human IgG. In the case of antigen detection, the control material can be a specific antigen. In a multi-analyte detection system, any one of the discrete areas can be used for a specific control if desired. In the case of a system which detects ANA profile, the discrete readable areas on the membrane have different antigens, for example Sci-70, SS-A(Ro), SS-B(La), Sm, Jo-1 and RNP immobilised and human IgG can be immobilised as a control. In an initial step of the test, the membrane is moistened with a wash/buffer containing a surfactant and a blocking agent, with the result that the membrane material is effectively rendered less hydrophobic and flow of test fluid and reagents across the membrane is facilitated. When a human serum containing antibodies against one or more of these antigens is added to the opening of the device, which may be in the form of a cassette, the antibodies in the sample will bind to a specific antigen on the solid support. The excess serum sample can then be removed by the addition of a few drops of a wash buffer. The excess liquid is captured underneath the membrane by the absorbent pad, keeping it away from the antigenic areas. Upon the addition of a few drops of a signal solution, a coloured round dot will appear where the reaction is positive. No coloured dots will appear when the reaction is negative. The control dot will always appear coloured to indicate that the reagents and device are functioning properly.

The invention also provides a method and apparatus for the detection in a sample of the presence or absence of antigenic substances, antibodies, or both or haptens using the above mentioned cassette device. It can be used for screening of the TORCH panel, for screening of a drugs of abuse panel, for auto-immune antibody screening, for gastroenterology profile screening (*H. Pylori,* Parital cells, LKM1, M2), HIV testing, *Micobacterium* screening and screening for infectious diseases in birds and animals.

### BRIEF DESCRIPTION OF THE DRAWINGS

Various embodiments of the invention will now be described, by way of example only, with reference to the accompanying drawings, in which:
Fig. 1 is an exploded view of a test cassette according to a first embodiment of the invention, Fig. 2 is a cross-section and Fig. 3 is a plan of the cassette;
Figs 4-9 are plan views of test cassettes according to further embodiments of the invention, provided with sample-receiving openings of different shape from that of Fig. 1 and suitable for testing more than two analytes;
Figs. 10a-10f show diagrammatically successive stages in an antibody screening test using the cassette of Figs 1-3; and
Figs. 11a and 11b are respectively a plan and a view in longitudinal section of another form of cassette.

### DETAILED DESCRIPTION OF PREFERRED EMBODIMENTS

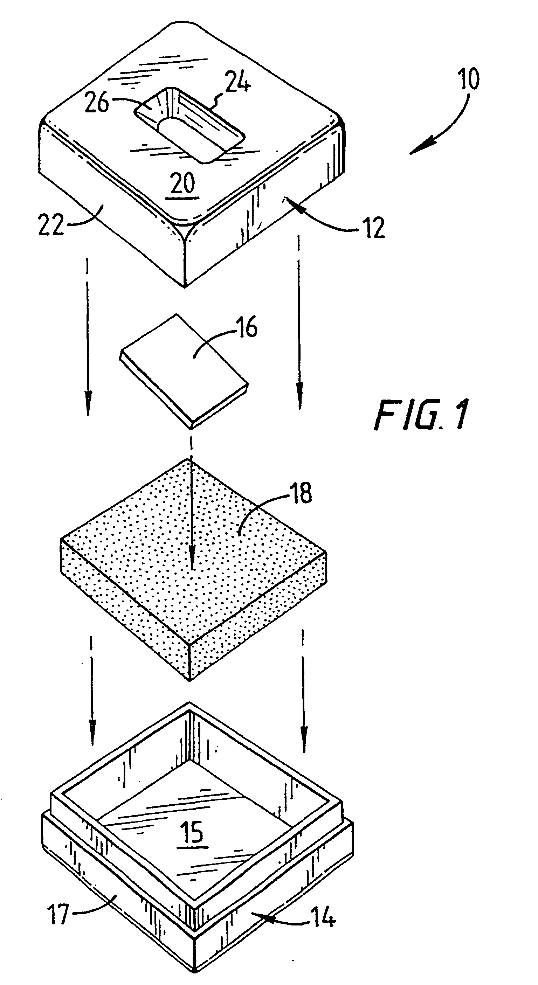

In Figure 1 there is shown a test cassette for use in immunological testing. The cassette is formed with a two part casing 10 comprising a lid 12, a tray 14, a relatively small sample-carrying membrane 16 that fits centrally beneath the lid 12, and a relatively large and thick adsorbent pad 18 that fits within the tray 14 beneath the membrane 16. The membrane 16 has a plurality of discrete readable areas which are spaced apart from one another. The readable areas will normally comprise at least one test area and at least one area providing a control. In each area there may be present an antigen or an antibody and the cassette may be used for sandwich assays or competitive assays.

The casing 10 may conveniently be of size about 3 cm by about 3 cm by about 1 cm. The lid 12 and tray 14 may be moulded of a plastics material, for example polystyrene, PVC or polypropylene. Size and shape can vary considerably depending on the application, but the present size and internal capacity has been found to be convenient for a number of applications. The material selected and wall thickness may be such as to impart an appropriate degree of rigidity to the assembled cassette, and typically the wall thickness may be about 1-2 mm. The lid and tray may be formed with inter-engageable formations (not shown) which releaseably snap fit together so that the parts may be assembled and so that after use the membrane carrying the results of a test can be removed and preserved as part of a patient's medical records.

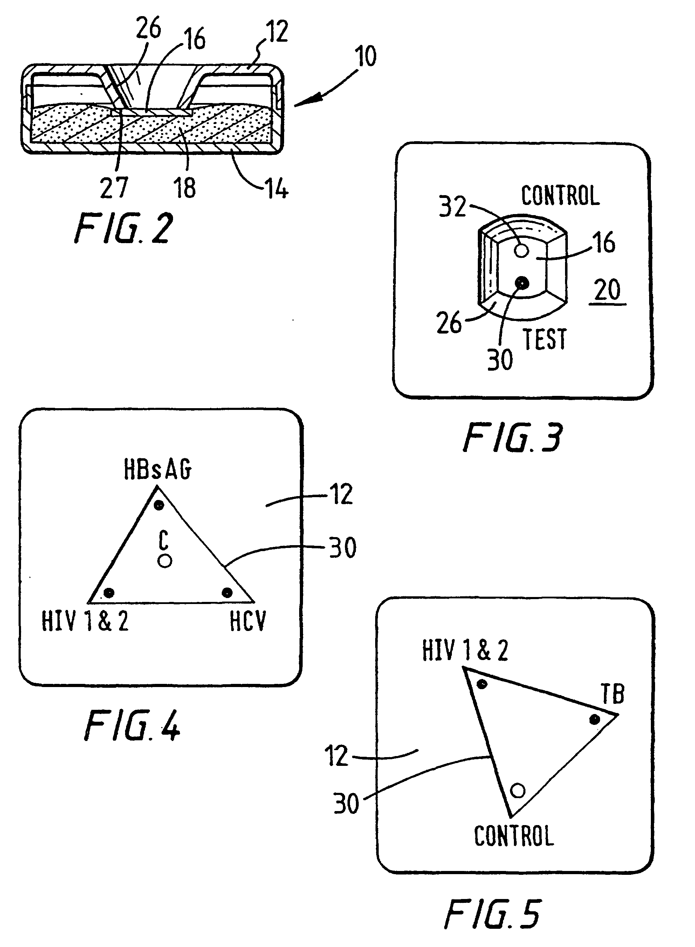

The lid 12 may be formed with a generally planar upper surface 20 bounded by depending peripheral walls 22 and a central opening 24 for admission of test fluid which in this embodiment is generally oval in plan and downwardly convergent in side view. The wall or walls 26 defining the central opening slope downwardly at about 45° which provides good aesthetics and provides a funnel or well structure which collects and provides containment for the test sample and other solutions used. Where the opening meets the pad, its length may be about 1 cm and its width may be about 0.5 cm. The wall 26 has a horizontally directed bluff lower end face 27 (Fig. 2) for holding and transmitting pressure to the membrane 16 so as to provide appropriate flow characteristics. The tray 14 conforms to the lid 12, has a base 15 and upstanding peripheral walls 17 which provide a butt or lap joint fit to the walls 22, and provides a receptacle for the body of absorbent material 18.

The dimensions ofthe affinity membrane 16 must be larger than those of the opening 24 and of the lower end of the wall 26, but usually only a small overlap is sufficient. In this embodiment the dimensions of the membrane may be 1.4 cm by 0.7 cm. It is used to provide discrete locations for the deposition and immobilisation of antigens, antibodies, polysaccharides, peptides, proteins or small molecules, for example drugs of abuse, or other materials to be employed in the test. The readable areas provided with test materials are located on the membrane 16 so that they appear through the opening 24, through which they can be treated simultaneously with test material and with reagents, and through which they can be read simultaneously.

The membrane 16 should have a water-retardant porous structure through which liquid flows gradually at a controlled rate so that liquid introduced into the opening 24 has a dwell-time before it passes through the membrane 16 and downwardly into the adsorbent pad 18. The dwell time will depend on the nature and viscosity of the liquid and, for example wash solutions and signal solutions will normally flow through more quickly because they contain surfactant and also because they do not contain as much protein and electrolyte as do patient samples. The assay conditions may be optimised for a dwell time of 60-90 seconds for the test sample which is appropriate for rapid testing which might give a result in 3-5 minutes, the wash and signal solutions which are less viscous typically running in 30-60 seconds. The membrane material should have a consistent and uniform pore size to give consistent flow rate and binding capacity for the antigen or antibody. It is preferably of a hydrophobic material so that droplets of immobilisation fluid e.g. of size 0.5 µl or 1 µl applied e.g. by a pipette tend not to spread out over its surface, and a porous nylon membrane material modified to contain reactive groups (e.g. -COOH groups and -NH₂ groups) has been found to be suitable. The hydrophobic/hydrophilic balance ofthe membrane is influenced by the presence of the reactive groups and should be such that aqueous liquids can flow through the capillary structure of the membrane, and it may be adjusted by impregnation of the membrane material with a surfactant. It will be appreciated that different membrane materials and physical characteristics may be used depending upon the test to be carried out. Other membrane materials which may be used include nitrocellulose, modified polysulfones, fibreglass, cellulose, polyethylene, polyvinylidine fluoride and the like having a pore size ranging from about 0.2 µm to 1.2 µm or larger, preferably from about 0.45 µm to 1.2 µm.

The absorbent pad 18 is selected to fit within the tray part and may be of a fibrous or spongy water-absorbent material. It should have an absorption capacity before saturation which is greater than the predetermined total liquid volume of the test procedure for which the cassette is designed. Its absorption capacity is preferably at least twice and often 5-10 times the total liquid volume so that liquid which has flowed into the absorbent material becomes dispersed away from the membrane and does not collect near it, which can give rise to reverse flow and consequently a high background colour. The depth of material in its uncompressed state should be slightly above the height from the bottom of the tray 14 to the base of the internal wall 26 so that the wall 26 compresses the absorbent material and holds the membrane against it in face to face contact so that flow of fluid through the membrane is facilitated. The material of the pad 18 may be of a hydrophilic fibrous or layered material, for example compressed cotton, or layers of filter paper or of other fibrous or layered cellulosic material. It may be an absorbent sponge of natural or synthetic polymer material which may work by the surface-tension induced "wicking" of aqueous liquid away from the membrane 16 into a fine porous structure, by having a greater affinity for aqueous liquid than the membrane 16 or by a combination of these two effects. It may be formed in two parts, with a layer of porous paper (e.g. filter paper) located immediately below and in contact with the membrane 16 to provide a strongly hydrophilic layer into which aqueous liquid from the membrane is drawn, and a body of sponge material behind the paper layer into whose capillary structure the liquid flows from the paper layer.

Various antibodies, antigens or haptens may be immobilised on the solid support or membrane 16, each in a discrete area which may be supplied with e.g 0.5-5 µl of a solution or suspension of the material to be tested which should be immobilised on the sample. Advantageously the molecular weight of the antibody, antigen or hapten may be increased by treatment with a cross-linking reagent to promote immobilisation and permit larger quantities of material to become immobilised in a given region of the membrane. Such cross-linking reagents include homo bi-functional, bi hetero-functional and multifunctional materials. Hetero-bifunctional cross-linkers may react, for example, with amino and thionyl groups. Cross-linking reagents that could be used include:
glutaraldehyde;
N-hydroxysuccinimide esters (NHS esters), e.g. sulfo-disuccinimidyl suberate (DSS);
Sulfo-NHS esters, e.g. sulfo-disuccinimidyl suberate (sulfo-DSS), dithiobis(succinimidyl propionate) (DSP), disulfosuccinimidyl tartarate (sulfo-DTS), and 4,4'-diisothiocyano-2,2'-stilbene disulfonic acid (DIDS);
Sulfosuccinimidyl 4-(N-maleimidomethyl)-cyclohexane-1-carboxylate (sulfo-SMCC);
m-maleimidobenzoyl-N-hydroxysulfosuccinimide ester (MBS) and sulfo-MBS;
Sulfosuccinimidyl 4-(p-maleimidophenyl) butyrate (sulfo-SMBP);
N-succinimidyl (4-iodoacetyl) aminobenzoate (SIAB)
N-succinimidyl 3-(2-pyridylthio) propionate (SPDP)
N-hydroxysuccinimidyl-4-azidosalicylic acid (NHS-ASA)
Sulfosuccinimidyl 6-(4-azido-2-nitrophenylamino)hexanoate (sulfo-SANPAH)

In order to give rise to a strongly observable response, immobilisation is at least partly by formation of a chemical bond between the test material and reactive groups of the membrane, either by direct reaction of the test material with reactive groups of the membrane material or by the use of a di- or polyfunctional linking reagent (as described above)which can react to form polymerised antigen which binds to the reactive groups of the membrane material Such chemical bonds permit that the high loadings of test material onto the test site can be achieved and maintained.

It is an advantage of the present multi-analyte detection system that it can be used to detect e.g. from 2 to 7 antigens simultaneously from a single sample within a period of about 3- 5 minutes. The present test may also be used to screen for drugs of abuse by competitive assay. Each drug may be conjugated to BSA and then immobilised in a separate discrete area on the membrane 16. When testing for HCG, LH and TSH, the test materials are immobilised with α-HCG antibodies. In a thyroid function test represented by TSH, T3, T4, Tg, etc, both antigen and antibodies may be immobilised on the membrane or support. The present cassette can also be used to screen for *Candida albicans, Chlamydia*, Syphilis, Hepatitis B and other infectious diseases. The present test can-be used to screen for gastroenterology (*H. Pylori,* Partial cells, LKM-1 and M2), HIV and a wide variety of infectious diseases in medical and veterinary practice. Examples of particular tests are given below:
(a) In the simplest case, shown in Fig.3, there are two test areas, one area 30 being for the test sample, where for example an antigen, antibody or hapten (e.g. a drug or hormone) can be immobilised, and the other area being for a control sample to indicate that the test is functioning properly and that the reagents used are performing as intended. The area 30 may be provided, for example with lipopolysaccharides representing a *Mycobacterium* antigen, a recombinant or synthetic protein which is a HIV1 or HIV2 antigenic substance, or an antigenic substance relating to *H. Pylori.* If the antibody being detected is human antibody against a test antigen or hapten, then the control area 32 may contain human IgG.
(b) Figures 4 and 5 show an alternative form of cassette in which the lid 12 is formed with a sample-receiving opening 30 of generally triangular shape which permits multi-analyte detection, but is otherwise of similar construction to the cassette of Figs 1-3. In the form shown in Fig. 4, the membrane 16 carries a centrally located control area C containing IgG and three test areas directed towards the vertices of the opening and in this instance carrying antigens related to HIV 1 & 2, HCV and HBsAG. In the alternative form shown in Fig 5, the cassette carries a control area and test areas for HIV 1 & 2 and for TB.

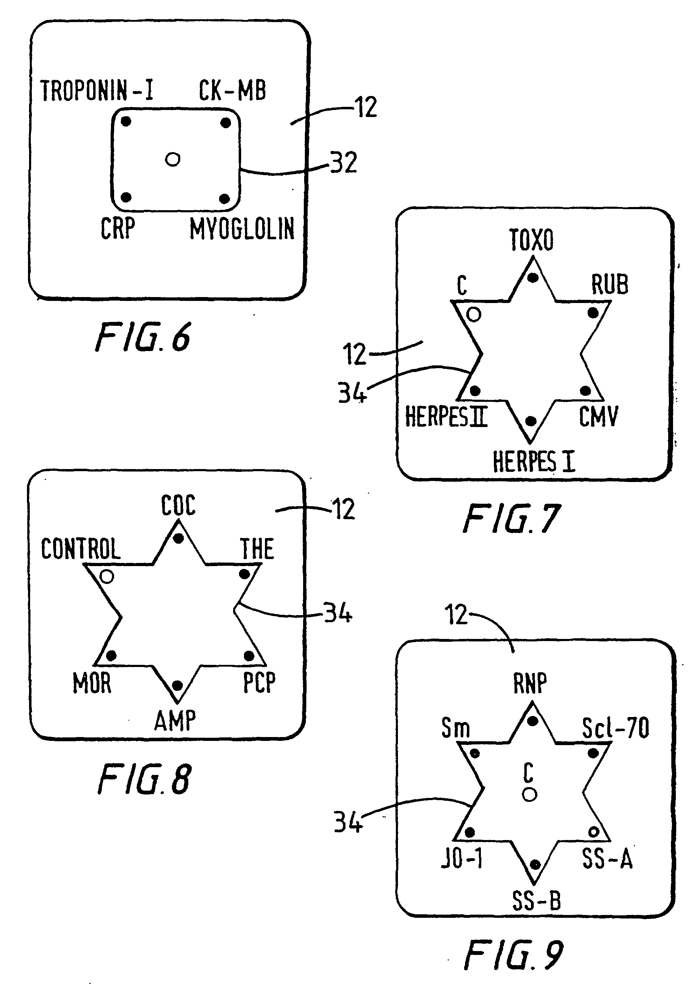

(c) Figure 6 shows a cassette generally as described with reference to Figs. 1-3 except that the sample-receiving opening 32 in the lid 12 is generally rectangular. In this instance there is a central control area C containing IgG, and four test areas directed towards the vertices of the rectangle and containing Troponin-I, CK-MB, CRP antigens and myglobin which can be used to test for myocardial infarction.
(d) Figs. 7-9 show cassettes generally as described with reference to Figs 1-3 except that the opening 34 in the lid 12 is star-shaped, the test areas being disposed towards the points of the star. In Fig 7 there is shown test areas for the TORCH panel screen including an IgG control antigen and antigens for *Toxoplasma gondii, Rubella,* CMV, *Herpes 1,* and *Herpes II.* Fig 8 shows a panel screen for the drugs of abuse tests (COC, THE, PCP, AMP and MOR antigens). In Fig. 9 there are shown test areas for the ANA screen for auto-immune diseases, the antigens immobilised in separate discrete areas being dDNA, RMP, Sm, SS-A(Ro), SS-B(La), Scl-70, JO-1 and SM, with a centrally located IgG control area C.

The present process and apparatus may also be used to detect antigens in a sample from a warm-blooded animal or from a bird. In this case the test areas have antibodies to the relevant antigens immobilised at the various test areas, and the signal solution may comprise a conjugate of an antigen with a visualising agent, for example an antigen-gold conjugate. The immobilisation procedure will differ from that for immobilisation of an antigen because of the lower molecular weights of antibodies (e.g. a HIV antigen which is only about 40 amino acids long), and commonly a bifunctional reagent e.g. glutaraldehyde may be used to form a bond (polymerisation) with the antibody or antigen that finally bind onto the membrane 16.

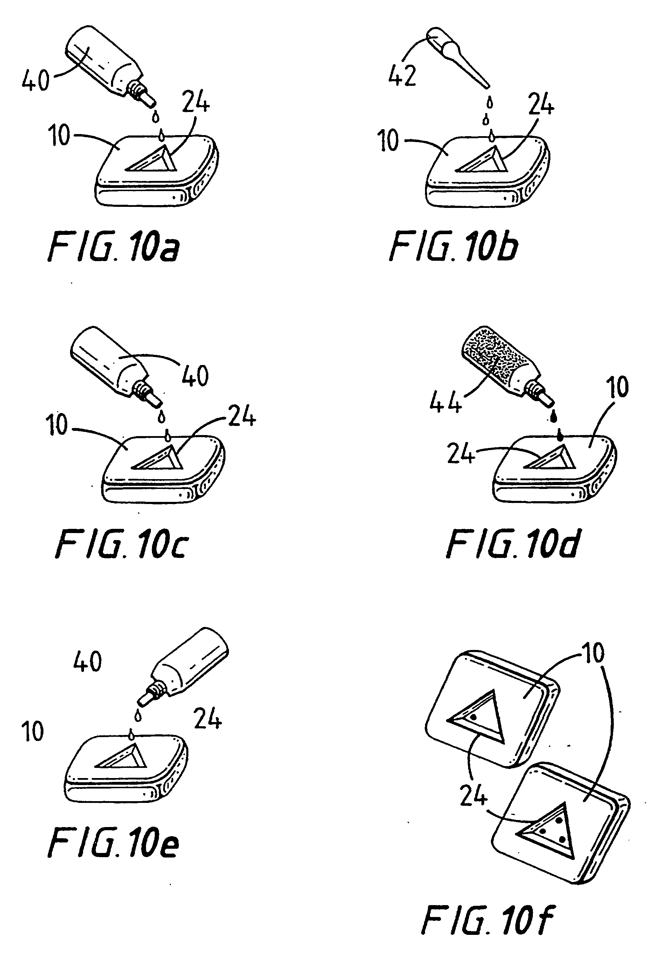

A "sandwich assay" procedure using the cassette will now be described with reference to Figs 10a-10f. In Fig 10a, a test is initiated by introducing 2 drops (80 µl) of wash buffer into the sample opening 24 of a cassette 10 from a dropper bottle 40. The wash/blocking solution present in the bottle 40 is used to block unbound sites on the membrane 16 and covers the whole of the membrane appearing through the sample opening and the test and control areas thereon, the same applying to the liquid added at subsequent stages in the test. A suitable wash/blocking solution may comprise:

| | |
|---|---|
| Ethanolamine | 0.1-10 wt % |
| Surfactant (Tween-20 or NP-40) | 10 wt % |
| Casein | 0.1-0.5 wt % |
| Phosphate buffer | 0.01-1M. |

Ethanolamine and casein both act as blocking agents, and Tween-20 also acts as a mild blocking agent as well as a surfactant. The ethanolamine in the above solution may be replaced by another amino-group containing compound. The solution will pass through the membrane in about 30 seconds.

A serum or plasma sample from a patient or from a test animal may be diluted e.g. 1:40 with the above mentioned wash buffer and 4 drops (160 µl) of the diluted patient serum is added to the opening 24 from a dropper 42 as shown in Fig 10b. Other samples may be tested, including urine, saliva, vaginal fluid, seminal fluid, inflammatory fluid, gastrointestinal fluid, pulmonary fluid, tissue extracts, whole blood or an extract derived by homogenisation of feces into a buffer and filtering.

Prior to dilution, the serum or other sample may have been pre-treated to remove non-specific binding proteins and improve the accuracy of the test results. After about 60 seconds the diluted patient sample should have passed through the membrane, and antibodies specific to the antigens on the solid support, if present, will form an antibody- antigen complex in the discrete test area and in the control area. A further two drops (80 µl) of the wash buffer is added from the dropping bottle 40 as shown in Fig. 10c in order to remove excess sample. The wash buffer can be expected to pass through the membrane 16 in about 30 seconds.

Three drops (120 µl) of signal solution may be added to the cassette 10 from a further dropping bottle 44 as shown in Fig 10d. A variety of different signal solutions may be used to make the test results visible to the naked eye or (less preferably) to a test instrument. Signal materials may include labelled immunologically active component such as hapten, antigen or antibody conjugated to:
(a) Aqueous dispersions of liposomes or other discrete colloidal particles comprised of a major portion of lipophilic compounds and a minor portion of immunologically active component (see US-A-4193983 and 4342826). The liposomes may be visibly labelled e.g. by the presence of a dye.
(b) Particles of an aqueous dispersion of (a) a hydrophobic dye or pigment or (b) polymer nuclei (see US-A-4373932). As indicated in the above patent the coloured organic compounds which are used as labels are hydrophobic and are insoluble in water or soluble only to a very limited extent. They are used in the form of a sol in which particles of the dye or pigment or of polymeric nuclei coated with the dye or pigment are dispersed in water and have a particle size of at least 5 nm and preferebly 10-500 nm.
(c) Non-diffusible cyan dye (see US-A-4419435).
(d) Colloidal metallic particles e.g. gold or silver.
(e) Latex particles e.g. of polystryene coated with antibody or antigen and having a colouring agent for visualisation (see e.g. US-A-5447837 at column 11).

Where the signal solution is based on the action of an enzyme (HRP, alkaline phosphatidase, glucose oxidase, galactosidase, etc), the corresponding chromatogen can be used to visualise the end of the reaction. In the present test, mono- or polyvalent polyclonal or monoclonal antibodies, Fab or Fc fractions of antibodies, fractions of antibodies, cloned or genetically engineered antibodies and the like may be used both as materials immobilised to the membrane and as immobilised components of the signal solution. For example, the signal solution may be formulated using the above antibodies conjugated to colloidal gold or other metallic particles (US-A-4407943), food dyes, enzymes, coloured beads or other visualising agents. A suitable signal solution comprises an antigen, for example Protein A or IgG, conjugated to 20 µm colloidal gold particles.

In a sandwich assay, the conjugate in the signal solution will bind to the control area and at the test area if an immuno-complex is formed with the test sample. A positive result is demonstrated by the appearance of a coloured dot in the relevant area, and the absence of a coloured dot in the relevant test area indicates the absence of a reaction with the sample and hence a negative result.

The colour of the dot will depend on the type of conjugate used. A colloidal gold conjugate of particle size about 20 µm will give pink to red coloured dots, whereas a blue colour will be obtained if the particle size is about 40 µm. A colloidal silver conjugate will give rise to dots which may range in colour from brown to black. If conjugated beads are used, the colour may be blue, red, black, etc, depending on that of the beads. A conjugated food dye will give a spot having the colour of the dye.

A period of about 60 seconds may be allowed for the signal solution to interact with the test and control areas on the membrane and to pass through the membrane, after which a further two drops (80 µl) of the wash buffer is added from the dropping bottle 40 as shown in Fig 10e in order to remove excess signal solution.. About 30 seconds are allowed for the wash buffer to pass through the membrane, after which the results are read, cassettes having the the appearance for a positive or negative result being shown in Fig 10f, no red dots indicating an invalid test, a single coloured dot showing in the liquid opening indicating a negative result and two coloured dots appearing in the liquid opening or well indicating a positive result. If the colour is insufficiently developed, additional signal solution may be added by repeating the procedures of Figs 10d and 10e. If washing has been insufficient, the entire membrane 16 may assume a light background colour, but this neither invalidates the test nor indicates a positive result. After the test is complete, the cassette 10 may be opened, and the membrane 16 may be removed, washed and dried to provide a permanent record of the test. This is desirably done on completion of the test or shortly afterwards because if the membrane 16 is left for extended periods in contact with a moist absorbent pad 18 containing test liquids and reagents, a background colour may develop or become strengthened.

The equipment necessary to conduct the above test may be provided as a pack which will include the cassette 10, bottles 40, 44 containing the wash buffer which may also be used to dilute the patient sample, and a dropper 42 for the patient sample. The test procedure set out above has a number of advantages:
(a) It is simple and inexpensive to use and may be completed within a short period, typically 5 minutes or less;
(b) Only two reagents are required, which can be pre-filled into dropping bottles 40,44 and no pipetting is required.
(c) The cassette 10 is disposible.
(d) The user can readily adjust the test conditions, e.g. by altering the volume of patient sample added and the volume of signal solution added, and a strong result can be obtained because relatively large volumes of signal solution can be flowed across the membrane 16..
(e) Depending upon the quality of the reagents used, a high degree of accuracy can be obtained in the test results.
(f) The result of the test may be directly observed, and no test instrument is required.
(g) Multiple analytes from a human or animal test subject may be measured from a single sample
(g) Highly trained personnel are not required, and the test can be used at home, in the field, or in a doctor's surgery, with a sample being obtained from the patient and used immediately. The test could be used on samples from athletes, for example, as a preliminary screen for drugs of abuse such as anabolic steroids. It may also be used under hospital or laboratory conditions with obtained samples being tested immediately or with samples that are tested after a delay e.g. because they have been delivered from a remote test site.

Various modifications may be made to the test procedure described above without departing from the invention. Small molecules or haptens can be detected if coated in discrete areas on the solid support or membrane 16. If desired, any of the discrete areas can be used to immobilise a negative control or standard material.

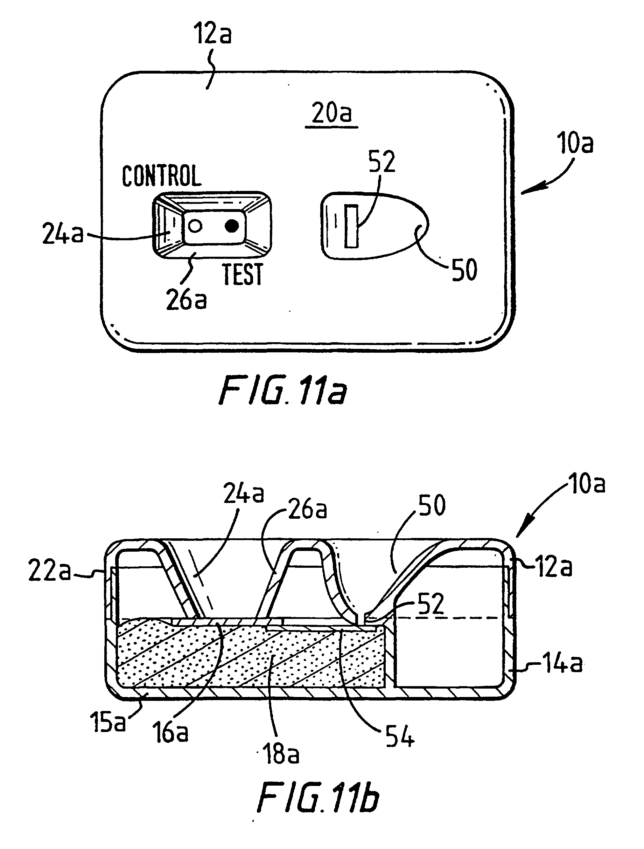

Figures 11a and 11b show a further form of the cassette particularly, though not exclusively, intended for the testing of serum from whole blood and which has provision both for producing a separation of the serum from the blood cells and other slow-moving blood fractions and for carrying out one or more immunochemical tests on the separated serum. The cassette comprises a two part casing 10a of length about 5cm, width about 3 cm and depth about 2 cm, said cassette comprising a lid 12a, a tray 14a, a relatively small sample-carrying affinity membrane 16a that fits centrally beneath the lid 12a, and a relatively large and thick adsorbent pad 18a that fits within the tray 14a beneath the membrane 16a.The lid 12a may be formed with a generally planar upper surface 20a bounded by depending peripheral walls 22a and a first opening 24a for admission of test fluid which in this embodiment is generally oval in plan and downwardly convergent in side view, although it may have a different shape e.g. as shown in Figs 4-9 described above. The wall or walls 26a defining the first opening slope downwardly at about 45° which provides good aesthetics and provides a funnel structure which collects and provides containment for the test sample and other solutions used. Where the opening meets the pad 16a, its length may be about 1 cm and its width may be about 0.5 cm. The wall 26a has a horizontally directed bluff lower end face for holding and transmitting pressure to the membrane 16a so as to provide appropriate flow characteristics for the test reagents which flow across the membrane as in the previous embodiments. The tray 14a conforms to the lid 12a, has a base 15a and provides a receptacle for the body of absorbent material 18a. Wash liquid and signal solution flow through the membrane from the opening 24a into the absorbent material 18a as in the cassette of Figs. 1-3. Blood is introduced through a second well 50 which leads to a second opening 52 through the lid 12a. A strip of porous material 54 leads from the opening 52 to the underside of the membrane 16a. Less mobile components of the blood are retarded by the porous material, so that plasma becomes separated and is supplied to the affinity membrane 16a, through which it flows laterally to each of the sample regions. Flow of blood to the sample and control regions is therefore through the porous structure of the affinity membrane 16a, whereas flow of wash liquid and visualising reagent is through the membrane, wherein the porosity and surface characteristics of the membrane are such that the liquid sample and the visualising solution take about 20-120 seconds to pass across the membrane.

The porous material 54 which acts as a plasma separator may be a sheet of an inert hydrophobic material such as fibreglass, nylon, nitrocellulose, polyvinylidine fluoride, polypropylene or an acetate type membrane and may have a pore size of 0.2-0.8 µm. It may be impregnated with a mixture of chemicals to facilitate the separation of plasma from the whole blood sample when applied at one side of the material surface. The thickness of the material 54 may be 20-250 µm. It may be impreganated with a mixture comprising:

| Aqueous PBS (pH 7.4) | |
|---|---|
| Sigma Poly Prep | 2 wt % |
| GAF Gantrez AN-139 | 8 wt % |
| Maltodextrose | 5 wt % |
| Anti-coagulant | qs |
| Anti-bacterial agent | 0.01 wt %. |

The impregnated sheet material 54 facilitates the separation of plasma components from a whole blood sample, and passes the plasma on to the adjacent testing membrane 16a located at the end of the plasma separator remote from the second opening 52.

Embodiments of the invention will now be further described with reference to the following examples.

### Example 1

### Detection of antibodies to Mycobacterium tuberculosis

A lipolysaccharide antigen is obtained from tuberculosis bacteria by chromatographic purification is provided in aqueous buffer and is concentrated to a strength of about 2 mg/ml. The solution is treated with 25% glutaraldehyde (10 µg/l) in water with vigorous mixing for about 30 minutes. The resulting cross-linked material is then suitable for immobilisation on a membrane support. As a control, purified human IgG is cross-linked in the same way.

An affinity membrane is provided in the form of a nylon sheet having an average pore size of about 0.45 µm and weight about 80g/m² and a portion about 14 mm by 7 mm is used. A test area is formed on the membrane by quantitatively applying 0.25- 1 µl of the polymerised antigen solution to a predetermined test site on the surface of the membrane and allowing it to dry for 5-30 minutes. A control area is formed in the same way on the membrane at a location adjacent to but about 2-4 mm apart from the test area. To complete the immobilisation of the control and test materials, the membrane is heated in an oven at 37°C for 15-30 minutes. During this period it is believed that covalent links between the active groups of the affinity membrane and the lipopolysaccharide antigen or the IgG are formed, and in addition that there are ionic, hydrophobic and hydrophilic interactions, all of which tend to hold the relevant material in place and increase the sensitivity of the test.

The affinity membrane with the test and control materials was used to make test cassette as described with reference to Figure 1. The external dimensions of the cassette (which was of thin walled polystyrene) were about 2.5 cm by 2.5 cm by 1 cm and the pad of absorbent material beneath the membrane was compressed cotton and had a liquid capacity of about 3 ml before it reached saturation. The pad was able to induce flow through the membrane which was pressed tightly against it for all the samples tested. Its holding capacity was several times the total capacity of the liquid required to perform the test, with the result that a strong unidirectional liquid flow was promoted and there was minimal flow-back and minimal background colour.

Blood was obtained from a number of patients with pulmonary TB and from people known to be free of tuberculosis. Serum was separated from the blood samples and tested according to the following procedure.

A cassette made as described above was wetted with two drops of a wash/blocking solution which is a phosphate buffered saline solution containing about 0.1 wt % ethanolamine, 0.05 wt % Tween 20 and 0.3 wt % casein. The casein in the wash solution serves to block the active sites of the affinity membrane during the initial wetting and stops antibodies or protein binding to those sites during the test which would otherwise colour the background. The wash solution typically takes 30 seconds to pass through the membrane into the underlying adsorbent material. The serum sample can be diluted 1:10 with the above wash/blocking solution and 3-4 drops are placed onto the membrane so that the membrane becomes covered with liquid which takes about 60-90 seconds to pass through the membrane. The membrane is then treated with two drops of the wash/block solution to wash off excess sample, followed by 2-3 drops of a signal solution to visualise the control area and the test area in the event of a positive response. The signal solution in this case is Protein A conjugated to 20 µm gold colloid. The control area should always become coloured red and the test area also becomes coloured red in the event of a positive response.

Results with a number of pulmonary TB patients gave close to 100 % specificity (i.e. all negative samples were found negative) and 85% sensitivity (samples found positive through this test as a proportion of those found positive through sputum culture). In every case the control area turned red. Repetition under identical conditions but without the use of glutaraldehyde as cross-linking agent was found to give significantly lower levels of response.

In place of the gold-based signal solution there may be used a signal solution based on a water-dispersible hydrophobic dye or pigment in which an immunochemically reactive component is coupled to particles of an aqueous dispersion of the dye or pigment, see for example US patent 4373932 and 4419435. With such dyes or pigments, the response colour of the test and control area may be varied at will e.g. to obtain a cyan or black response. There may also be used colloids of transition metals other than gold.

### Example 2

### Detection of antibodies to HIV 1 & 2 antigen

The procedure of Example 1 was repeated except that
(a) The test site was coated with a solution made by cross-linking with glutaraldehyde a recombinant HIV-1 protein mixed with HIV-2(rod) synthetic peptide.
(b) Sensitivity was checked by testing the serum with an FDA-approved ELISA and Western Blot assay.

Specificity was found to be 100% and sensitivity was found on the basis of 500 patient samples and compared to Western Blot assay to be 100%.

The procedure was repeated using saliva samples from a limited number of patients, and similar specificity and sensitivity was obtained.

### Example 3

### Detection of antibodies to H. Pylori antigen

The procedure of Example 1 was followed except that the test site was treated with an *H*. *Pylori* antigen. Specificity was checked by obtaining a biopsy and culturing the resulting sample. In a trial involving some 300 patients specificity was 98% and sensitiviity was 97%.

## Claims

1. Apparatus for performing an immunological test comprising:
(a) a membrane having first and second faces between which aqueous liquid can flow and having a microporous structure permitting aqueous liquid to flow gradually from one face to the other;
(b) a plurality of readable areas spaced apart from one another across the membrane and each having a respective antigen, antibody or hapten immobilised thereon;
(c) a cover for a first face of the membrane and formed with means defining a first well for supply of aqueous liquid to the membrane, the readable areas being visible through the first well and being arranged for simultaneous contact with aqueous liquid supplied into the first well;
(d) a second well formed in the cover spaced from the first well;
(e) a plasma separator of porous material leading from said second well and having a portion adjacent the membrane; and
(f) absorbent material in contact with the second face of the membrane for reception of aqueous liquid which has flowed across the membrane, there being tight contact between the first face of the membrane and the first well, and between the second face of the membrane and the absorbent material.

2. An apparatus according to claim 1, wherein the plasma separator is a strip of porous material.

3. An apparatus according to claim 1 or claim 2, wherein the plasma separator is formed from an inert hydrophobic material.

4. An apparatus according to claim 3, wherein the inert hydrophobic material is fibreglass, nylon, nitro cellulose, polyvinylidene fluoride, polypropylene or acetate.

5. An apparatus according to any preceding claim, wherein the plasma separator has a pore size of from 0.2 to 0.8 µm.

6. An apparatus according to any preceding claim wherein the plasma separator is impregnated with a composition to facilitate separation of serum from a whole blood sample placed in the second well.

7. An apparatus according to claim 6, wherein the composition comprises:
| Aqueous PBS (pH 7.4) | |
|---|---|
| Sigma Poly Prep | 2 wt % |
| GAF Gantrez AN-139 | 8 wt % |
| Maltodextrose | 5 wt % |
| Anti-coagulant | qs |
| Anti-bacterial agent | 0.01 wt %. |

8. The apparatus according to any preceding claim, wherein the membrane is of a hydrophobic material modified to contain reactive groups.

9. The apparatus of any preceding claim, wherein the membrane is of nylon.

10. The apparatus of any preceding claim, wherein the membrane has a pore size of about 0.2-1.2 µm.

11. The apparatus of any preceding claim, wherein the membrane has a pore size of about 0.45 µm.

12. The apparatus of any preceding claim, wherein at least one of the readable areas is for detection of antibodies to *Mycobacterium tuberculosis.*

13. The apparatus of any of claims 1-11, wherein at least one of the readable areas is for the detection of antibodies to HIV 1 and/or 2.

14. The apparatus of any of claims 1-11, wherein at least one of the readable areas is for the detection of antibodies to *H. Pylori.*

15. The apparatus of any of claims 1-11, wherein there are readable areas providing a panel test for the detection of antibodies arising as a result of myocardial infarction, for a TORCH panel screen, for gastroenterology screening, for the ANA screen, or for screening a panel of drugs of abuse.

16. The apparatus of any preceding claim, wherein the test areas comprise antibodies or antigens which have been subjected to cross-linking.

17. The apparatus of any preceding claim, wherein the antibodies, antigens or haptens are immobilised on the readable areas at least partly by the formation of covalent bonds to the membrane.

18. The apparatus of any preceding claim, wherein there are two readable areas appearing through the well.

19. The apparatus of any of claims 1-17, wherein there are at least three readable areas appearing through the well.

20. The apparatus of any preceding claim, wherein the absorbent material is cotton or paper.

21. The apparatus of any preceding claim for use in a test involving a predetermined total volume of aqueous liquid, wherein the absorption capacity of the absorbent material is a multiple of the volume of the test liquid.

22. The apparatus of claim 21, wherein the absorption capacity of the absorbent material is at least 5 times the total volume of the aqueous liquid.

23. The apparatus of claim 21, wherein the absorption capacity of the absorbent material is at least 10 times the total volume of the aqueous liquid.

24. The apparatus of any preceding claim, wherein the absorbent material is resilient and urges the membrane into contact with the well.

25. A test kit comprising the apparatus of any preceding claim, a dispensing container for a wash/buffer solution and a dispensing container for a visualising agent.

26. The test kit of claim 25, wherein the wash/buffer solution and/or the visualising agent contain a surfactant.

27. A method of carrying out an immunological test which comprises providing apparatus as claimed in any of claims 1 to 24 or a kit as claimed in claim 25 or claim 26, introducing a liquid sample to be tested into the first well and allowing it to pass across the membrane, introducing a developing or visualising solution into the first well and allowing it to pass across the membrane to develop an observable result.

28. A method of carrying out an immunological test which comprises providing an apparatus according to any of claims 1 to 24 or a kit as claimed in claim 25 or claim 26, introducing a whole blood sample into the second well and allowing plasma from the sample to pass via the plasma separator to the membrane, and introducing a developing or visualising solution into the first well and allowing the solution to pass across the membrane to develop an observable result.

29. A method according to claim 27 or claim 28, wherein a wash/buffer containing a surfactant is applied to the membrane before the liquid or blood sample is introduced.

30. A method according to claim 27, wherein the porosity and surface characteristics of the membrane are such that the liquid sample and the visualising solution take about 20-120 seconds to pass across the membrane.

31. A method according to claim 27, which is a sandwich assay.

32. A method according to claim 27, which is a competitive assay.

33. A method according to any of claims 27-32, wherein the visualising solution comprises a visible dye or pigment.

## Patentansprüche

1. Vorrichtung zur Durchführung eines immunologischen Tests mit:
(a) einer Membran mit einer ersten und zweiten Seite, zwischen denen wäßrige Flüssigkeiten fließen können, und die eine mikroporöse Struktur aufweist, die eine wäßrige Flüssigkeit von einer zur anderen Seite hindurchsickern läßt;
(b) mehreren Ablesezonen, die voneinander getrennt über die Membran verteilt sind und jeweils entweder Antigen, Antikörper oder Hapten in immobilisiertem Zustand aufweisen;
(c) einer Abdeckung für die erste Membranseite, in der ein Reservoir zum Liefern wäßriger Flüssigkeit an die Membran ausgebildet ist, wobei die Ablesezonen durch das erste Reservoir hindurch sichtbar angeordnet sind und zwar so, daß sie gleichzeitig mit der im ersten Reservoir befindlichen wäßrigen Flüssigkeit in Kontakt stehen;
(d) einem zweiten Reservoir, das in einem Abstand zum ersten Reservoir in der Abdeckung ausgebildet ist;
(e) einem Plasmaabscheider aus porösem Material, der vom zweiten Reservoir ausgeht und einen an die Membran angrenzenden Abschnitt aufweist; und
(f) einem Absorptionsmaterial, das die zweite Seite der Membran berührt, um die wäßrige Flüssigkeit, die durch die Membran geflossen ist, aufzunehmen, wobei zwischen der ersten Membranseite und dem ersten Reservoir und zwischen der zweiten Membranseite und dem Absorptionsmaterial ein enger Kontakt besteht.

2. Vorrichtung nach Anspruch 1, wobei der Plasmaabscheider ein Streifen aus porösem Material ist.

3. Vorrichtung nach Anspruch 1 oder 2, wobei der Plasmaabscheider aus einem inerten, hydrophoben Material gebildet ist.

4. Vorrichtung nach Anspruch 3, wobei das inerte hydrophobe Material entweder Fiberglas, Nylon, Nitrozellulose, Polyvinylidenfluorid, Polypropylen oder Acetat ist.

5. Vorrichtung nach einem der vorhergehenden Ansprüche, wobei der Plasmaabscheider eine Porengröße von 0,2 bis 0,8 µm aufweist.

6. Vorrichtung nach einem der vorhergehenden Ansprüche, wobei der Plasmaabscheider mit einer Zusammensetzung imprägniert ist, um aus einer im zweiten Reservoir befindlichen Vollblutprobe Serum abzuscheiden.

7. Vorrichtung nach Anspruch 6, wobei die Zusammensetzung umfaßt:
| Wäßrige PBS (pH 7,4) | |
|---|---|
| Sigma Poly Prep | 2 Gew.-% |
| GAF Gantrez AN-139 | 8 Gew.-% |
| Maltodextrose | 5 Gew.-% |
| Anticoagulant | in genügender Menge |
| Antibakterielles Mittel | 0,01 Gew.-% |

8. Vorrichtung nach einem der vorhergehenden Ansprüche, wobei die Membran aus hydrophobem Material besteht, das so verändert ist, daß es reaktive Gruppen enthält.

9. Vorrichtung nach einem der vorhergehenden Ansprüche, wobei die Membran aus Nylon besteht.

10. Vorrichtung nach einem der vorhergehenden Ansprüche, wobei die Membran eine Porengröße von etwa 0,2 bis 1,2 µm aufweist.

11. Vorrichtung nach einem der vorhergehenden Ansprüche, wobei die Membran eine Porengröße von etwa 0,45 µm aufweist.

12. Vorrichtung nach einem der vorhergehenden Ansprüche, wobei mindestens eine der Ablesezonen für die Erkennung von Antikörpern zum *Mycobacterium Tuberculosis* vorgesehen ist.

13. Vorrichtung nach einem der Ansprüche 1 bis 11, wobei mindestens eine der Ablesezonen für die Erkennung von HIV 1- und/oder 2-Antikörpern vorgesehen ist.

14. Vorrichtung nach einem der Ansprüche 1 bis 11, wobei mindestens eine der Ablesezonen für die Erkennung von Antikörpern zu *H. Pylori* vorgesehen ist.

15. Vorrichtung nach einem der Ansprüche 1 bis 11, wobei die Ablesezonen einen repräsentativen "Panel Test" zur Erkennung von Antikörpern liefern, die als Folge eines myokardialen Infarkts entstehen, für einen repräsentativen TORCH-Test, für einen gastroenterologischen Test, für den ANA-Test oder zum Testen eines Querschnitts von Mißbrauchsdrogen.

16. Vorrichtung nach einem der vorhergehenden Ansprüche, wobei die Testzonen vernetzte Antikörper oder Antigene aufweisen.

17. Vorrichtung nach einem der vorhergehenden Ansprüche, wobei die Antikörper, Antigene oder Haptene in den Ablesezonen mindestens durch eine teilweise Ausbildung kovalenter Bindungen an die Membran immobilisiert sind.

18. Vorrichtung nach einem der vorhergehenden Ansprüche, wobei zwei Ablesezonen durch das Reservoir hindurch sichtbar sind.

19. Vorrichtung nach einem der Ansprüche 1 bis 17, wobei mindestens drei Ablesezonen durch das Reservoir hindurch sichtbar sind.

20. Vorrichtung nach einem der vorhergehenden Ansprüche, wobei das Absorptionsmaterial Baumwolle oder Papier ist.

21. Vorrichtung nach einem der vorhergehenden Ansprüche zur Verwendung bei einem Test mit einem bestimmten Gesamtvolumen an wäßriger Flüssigkeit, wobei die Absorptionskapazität des Absorptionsmaterials ein Vielfaches des Testflüssigkeitsvolumens ist.

22. Vorrichtung nach Anspruch 21, wobei die Absorptionskapazität des Absorptionsmaterials mindestens das Fünffache des Gesamtvolumens der wäßrigen Flüssigkeit beträgt.

23. Vorrichtung nach Anspruch 21, wobei die Absorptionskapazität des Absorptionsmaterials mindestens das Zehnfache des Gesamtvolumens der wäßrigen Flüssigkeit beträgt.

24. Vorrichtung nach einem der vorhergehenden Ansprüche, wobei das Absorptionsmaterial elastisch ist und die Membran an das Reservoir drückt.

25. Test-Satz, der die Vorrichtung nach einem der vorhergehenden Ansprüche, einen Abgabebehälter für eine Wasch/Puffer-lösung und einen für ein Darstellungsmittel umfaßt.

26. Test-Satz nach Anspruch 25, wobei die Wasch/Pufferlösung und/oder das Darstellungsmittel ein Tensid enthält.

27. Verfahren zur Durchführung eines immunologischen Tests mit den Schritten: Bereitstellen einer Vorrichtung nach einem der Ansprüche 1 bis 24 oder eines Satzes nach Anspruch 25 oder 26, Einbringen einer zu testenden Flüssigkeitsprobe in das erste Reservoir und durch die Membran Passierenlassen, Einbringen einer Entwicklungs- oder Darstellungslösung in das erste Reservoir und durch die Membran Passierenlassen, um ein ablesbares Ergebnis zu entwickeln.

28. Verfahren zum Durchführen eines immunologischen Tests mit den Schritten: Bereitstellen einer Vorrichtung nach einem der Ansprüche 1 bis 24 oder eines Satzes nach Anspruch 25 oder 26, Einbringen einer Vollblutprobe in das zweite Reservoir und Passierenlassen des Plasmas durch den Plasmaabscheider an die Membran, und Einbringen einer Entwicklungsoder Darstellungslösung in das erste Reservoir und Passierenlassen der Lösung durch die Membran, um ein ablesbares Ergebnis zu entwickeln.

29. Verfahren nach einem der Ansprüche 27 oder 28, wobei eine Wasch/Pufferlösung mit einem Tensid auf die Membran aufgebracht wird, bevor die Flüssigkeits- oder Blutprobe eingebracht wird.

30. Verfahren nach Anspruch 27, wobei die Porosität und die Oberflächeneigenschaften der Membran so sind, daß die Flüssigkeitsprobe und die Darstellungslösung etwa 20-120 Sekunden benötigen, um die Membran zu passieren.

31. Verfahren nach Anspruch 27, bei dem es sich um einen Sandwich-Assay handelt.

32. Verfahren nach Anspruch 27, bei dem es sich um einen kompetitiven Test handelt.

33. Verfahren nach einem der Ansprüche 27 bis 32, wobei die Darstellungslösung einen sichtbaren Farbstoff oder ein Pigment umfaßt.

## Revendications

1. Dispositif pour réaliser un test immunologique, comprenant :
(a) une membrane ayant une première et une seconde faces entre lesquelles un liquide aqueux peut s'écouler et ayant une structure microporeuse permettant au liquide aqueux de s'écouler progressivement de l'une des faces jusqu'à l'autre ;
(b) une pluralité de surfaces pouvant être lues espacées d'une certaine distance les unes des autres sur la membrane et portant chacune un antigène, un anticorps ou un haptène respectif immobilisé sur celles-ci ;
(c) un couvercle pour une première face de la membrane et formée avec un moyen définissant un premier puits pour fournir un liquide aqueux à la membrane, les surfaces pouvant être lues étant visibles à travers le premier puits et étant disposées de façon à assurer un contact simultané avec le liquide aqueux fourni dans le premier puits ;
(d) un second puits formé dans le couvercle à une certaine distance du premier puits ;
(e) un séparateur de plasma de matériau poreux partant dudit second puits et ayant une partie adjacente à la membrane ;
(f) un matériau absorbant en contact avec la seconde face de la membrane pour la réception du liquide aqueux qui s'est écoulé à travers la membrane, un contact étroit étant assuré entre la première face de 1a membrane et le premier puits, ainsi qu'entre la seconde face de la membrane et le matériau absorbant.

2. Dispositif suivant la revendication 1, dans lequel le séparateur de plasma est une bande de matériau poreux.

3. Dispositif suivant la revendication 1 ou la revendication 2, dans lequel le séparateur de plasma est formé à partir d'un matériau hydrophobe inerte.

4. Dispositif suivant la revendication 3, dans lequel le matériau hydrophobe inerte est une fibre de verre, du nylon, de la nitrocellulose, du fluorure de polyvinylidène, du polypropylène ou un acétate.

5. Dispositif suivant l'une quelconque des revendications précédentes, dans lequel le séparateur de plasma est a une dimension de pore de 0,2 à 0,8 µm.

6. Dispositif suivant l'une quelconque des revendications précédentes dans lequel le séparateur de plasma est imprégné d'une composition pour faciliter 1 séparation du sérum d'un échantillon de sang entier placé dans le second puits.

7. Dispositif suivant la revendication 6, dans lequel la composition comprend :
| PBS aqueux (pH 7,4) | |
|---|---|
| Sigma Poly Prep | 2% en poids |
| GAF Gantrez AN-139 | 8% en poids |
| Maltodextrose | 5% en poids |
| Anticoagulant | q.s. |
| Agent antibactérien | 0,01% en poids |

8. Dispositif suivant l'une quelconque des revendications précédentes, dans lequel la membrane est constituée d'un matériau hydrophobe modifié de façon à contenir des groupes réactifs.

9. Dispositif suivant l'une quelconque des revendications précédentes, dans lequel la membrane est en nylon.

10. Dispositif suivant l'une quelconque des revendications précédentes, dans lequel la membrane a une dimension de pore d'environ 0,2 à 1,2 µm.

11. Dispositif suivant l'une quelconque des revendications précédentes, dans lequel la membrane a une dimension de pore d'environ 0,45 µm.

12. Dispositif suivant l'une quelconque des revendications précédentes, dans lequel l'une au moins des surfaces pouvant être lues est destinée à la détection d'anticorps dirigés contre *Mycobacterium tuberculosis.*

13. Dispositif suivant l'une quelconque des revendications 1 à 11, dans lequel l'une au moins des surfaces pouvant être lues est destinée à la détection d'anticorps dirigés contre les VIH 1 et/ou 2.

14. Dispositif suivant l'une quelconque des revendications 1 à 11, dans lequel l'une au moins des surfaces pouvant être lues est destinée à la détection d'anticorps dirigés contre *H. Pylori.*

15. Dispositif suivant l'une quelconque des revendications 1 à 11, dans lequel il existe des surfaces pouvant être lues fournissant un test de panel pour la détection d'anticorps résultant d'un infarctus du myocarde, pour un criblage de panel du syndrome TORCH, pour un dépistage en gastro-entérologie, pour le criblage ANA, ou pour le dépistage d'un panel de drogues entraînant une pharmacodépendance.

16. Dispositif suivant l'une quelconque des revendications précédentes, dans lequel les surfaces de test comprennent des anticorps ou des antigènes qui ont été soumis à une réticulation.

17. Dispositif suivant l'une quelconque des revendications précédentes, dans lequel les anticorps, les antigènes ou les haptènes sont immobilisés sur les surfaces pouvant être lues au moins partiellement par la formation de liaisons covalentes avec la membrane.

18. Dispositif suivant l'une quelconque des revendications précédentes, dans lequel il existe deux surfaces pouvant être lues qui apparaissent à travers le puits.

19. Dispositif suivant l'une quelconque des revendications 1 à 17, dans lequel il existe au moins trois surfaces pouvant être lues qui apparaissent à travers le puits.

20. Dispositif suivant l'une quelconque des revendications précédentes, dans lequel le matériau absorbant est du coton ou du papier.

21. Dispositif suivant l'une quelconque des revendications précédentes, utile dans un test impliquant un volume total prédéterminé de liquide aqueux, dans lequel la capacité d'absorption du matériau absorbant est un multiple du volume du liquide de test.

22. Dispositif suivant la revendication 21, dans lequel la capacité d'absorption du matériau absorbant est au moins 5 fois le volume total du liquide aqueux.

23. Dispositif suivant la revendication 21, dans lequel la capacité d'absorption du matériau absorbant est au moins 10 fois le volume total du liquide aqueux.

24. Dispositif suivant l'une quelconque des revendications précédentes, dans lequel le matériau absorbant est élastique et presse la membrane en contact avec le puits.

25. Nécessaire de test comprenant un dispositif suivant l'une quelconque des revendications précédentes, un récipient de distribution pour une solution de lavage/tampon et un récipient de distribution pour un agent de visualisation.

26. Nécessaire de test suivant la revendication 25, dans lequel la solution de lavage/tampon et/ou l'agent de visualisation contiennent un tensioactif.

27. Procédé pour réaliser un test immunologique, qui comprend la fourniture d'un dispositif suivant l'une quelconque des revendications 1 à 24 ou d'un nécessaire suivant la revendication 25 ou la revendication 26, l'introduction d'un échantillon liquide à tester dans le premier puits et sa traversée de la membrane, l'introduction d'une solution de développement ou de visualisation dans le premier puits et sa traversée de la membrane pour développer un résultat observable.

28. Procédé pour réaliser un test immunologique qui comprend la fourniture d'un dispositif suivant l'une quelconque des revendications 1 à 24 ou d'un nécessaire suivant la revendication 25 ou la revendication 26, l'introduction d'un échantillon de sang entier dans le second puits et la traversée du plasma de l'échantillon par l'intermédiaire du séparateur de plasma jusqu'à la membrane, et l'introduction d'une solution de développement ou de visualisation dans le premier puits et la traversée de la solution de la membrane pour développer un résultat observable.

29. Procédé suivant la revendication 27 ou la revendication 28, dans lequel un liquide de lavage/tampon contenant un tensioactif est appliqué à la membrane avant l'introduct de l'échantillon de liquide ou de sang.

30. Procédé suivant la revendication 27, dans lequel la porosité et les caractéristiques de surface de la membrane sont telles que l'échantillon de liquide et la solution de visualisation prennent environ 20 à 120 secondes pour traverser la membrane.

31. Procédé suivant la revendication 27, qui est un essai sandwich.

32. Procédé suivant la revendication 27, qui est un essai de compétition.

33. Procédé suivant l'une quelconque des revendications 27 à 32, dans lequel la solution de visualisation comprend un colorant ou un pigment visible.
